# EUROPEAN PATENT APPLICATION

(11) **EP 4 394 372 A1**
(43) Date of publication of application: **03.07.2024**
(21) Application number: 22860073.0
(22) Date of filing: 08.07.2022
(51) Int. Cl.: G01N 30/06, G01N 30/02, G01N 30/72

(54) **METHOD FOR TREATING BLOOD PLASMA SAMPLE CONTAINING CLOPIDOGREL OXIDE, AND MEASUREMENT METHOD**

(30) Priority: 23.08.2021 CN 202110967592
(71) Applicant: Chengdu Shibeikang Biomedical Technology Co., Ltd., Chengdu, Sichuan 611731 (CN)
(72) Inventor: ZHANG, Hai, Chengdu, Sichuan 611730 (CN); MOU, Xia, Chengdu, Sichuan 611730 (CN)
(74) Representative: Herrero & Asociados, S.L.
(86) International application number: PCT/CN2022/104622
(87) International publication number: WO 2023/024727

(57) **Abstract**

The disclosure discloses a method for treating a plasma sample containing (7aS,2'S)-2-oxo-clopidogrel and a method for determining the same, which belongs to the technical field of pharmaceutical analysis and solves the problem in the prior art that there is not yet a method for determining the concentration of (7aS,2'S)-2-oxo-clopidogrel in plasma. The method for treating a plasma sample containing (7aS,2'S)-2-oxo-clopidogrel comprises: taking whole blood containing (7aS,2'S)-2-oxo-clopidogrel, adding a reducing agent solution and collecting plasma. The method for determining (7aS,2'S)-2-oxo-clopidogrel in plasma according to the disclosure uses the treatment method as described above to treat a sample to be determined, and includes the following steps: S1. Whole blood treatment: taking whole blood, adding a reducing agent solution, centrifuging, and collecting plasma; S2. Plasma sample pre-treatment: adding a protein precipitant and an internal standard working solution to the plasma, vortexing and then centrifuging, and taking the supernatant; S3. LC/MS/MS determination. The method of the disclosure is simple, easy to operate, and has good stability, specificity and sensitivity, and accurate determination results.

## Description

### Field of Technology

The disclosure belongs to the technical field of pharmaceutical analysis, and specifically relates to a method for treating a plasma sample containing (7aS,2'S)-2-oxo-clopidogrel and a method for determining the same.

### Background of Art

Clopidogrel is a thienopyridine derivative, which itself is an inactive prodrug that requires a two-step metabolism in the body to produce an active thiol derivative (R-130964). Phase I: clopidogrel is metabolized by CYP1A2 (-36%), CYP2B6 (-19%), CYP2C19 (-45%), or the like, into inactive 2-oxo-clopidogrel; Phase II: 2-oxo-clopidogrel is further metabolized into the active thiol derivative (R-130964).

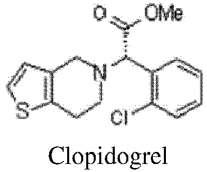

Because clopidogrel needs to undergo a two-step metabolic process to produce the active substance, it takes effect slowly. In the prior art, a solution of using its primary metabolite (7aS, 2'S)-2-oxo-clopidogrel as a drug is proposed.

(7aS,2'S)-2-oxo-clopidogrel, chemical name methyl (S)-2-(2-chlorophenyl)-2-((S)-2-oxo-2,6,7,7a-tetrahydrothieno[3,2-c]pyridin-5(4H)-yl)acetate, has the following structural formula:

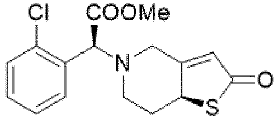

In the prior art, there is no method for the determination of plasma concentration of (7aS,2'S)-2-oxo-clopidogrel. Therefore, it is an urgent problem to be solved by those skilled in the art to provide a method for determining (7aS,2'S)-2-oxo-clopidogrel in plasma, with high specificity, high sensitivity and good stability.

### Summary of the Invention

One object of the disclosure is to provide a method for treating a plasma sample containing (7aS,2'S)-2-oxo-clopidogrel, which is simple, easy to handle and has good stability.

Another object of the disclosure is to provide a method for determining (7aS,2'S)-2-oxo-clopidogrel in plasma, comprising treating a sample to be determined, by using the above treatment method.

In order to achieve the above objects, the disclosure has adopted the technical solutions as follows.

The disclosure provides a method for treating a plasma sample containing (7aS,2'S)-2-oxo-clopidogrel, comprising: taking whole blood containing (7aS,2'S)-2-oxo-clopidogrel, adding a reducing agent and collecting plasma.

In some embodiments of the disclosure, the reducing agent is a protein reducing agent; preferably, the protein reducing agent is at least one of dithiothreitol, tris(2-carboxyethyl)phosphine, and 2-mercaptoethanol, more preferably tris(2-carboxyethyl)phosphine.

Preferably, a reducing agent solution is added, and the concentration of the reducing agent solution is 50-500 mg/ml.

Preferably, the concentration of the reducing agent in the plasma is 10-100 mg/ml.

The disclosure has unexpectedly found that the stability of (7aS,2'S)-2-oxo-clopidogrel in the plasma can be effectively improved by adding the protein reducing agent.

In some embodiments of the disclosure, when commercially available blood collection tubes are used to take the whole blood, since an anticoagulant is already included in the blood collection tubes, the reducing agent solution is added after the whole blood has been anticoagulated.

The disclosure provides a method for determining (7aS,2'S)-2-oxo-clopidogrel in plasma, comprising treating a sample to be determined, by using the treatment method as described above.

In some embodiments of the disclosure, LC/MS/MS is used for determining (7aS,2'S)-2-oxo-clopidogrel in plasma, and the method includes the following steps:
S1. Whole blood treatment: taking whole blood, adding a reducing agent solution, centrifuging, and collecting plasma;
S2. Plasma sample pre-treatment: adding a protein precipitant and an internal standard working solution to the plasma, vortexing and then centrifuging, and taking the supernatant;
S3. LC/MS/MS determination: injecting the supernatant obtained from step S2 into an ultra-high performance liquid chromatography tandem mass spectrometry for determination, wherein the liquid chromatography conditions include: a chromatographic column with octadecylsilane bonded silica gel packing, acetonitrile as mobile phase A and aqueous formic acid solution as mobile phase B, and a gradient elution is carried out as specified below:
   0 min, 5-15% A, 85-95% B;
   2 min, 50-60% A, 40-50% B;
   5 min, 50-60% A, 40-50% B;
   6 min, 85-95% A, 5-15% B;
   8 min, 85-95% A, 5-15% B;
   8.5 min, 5-15% A, 85-95% B;
   10 min, 5-15% A, 85-95% B;
   mass spectrometry conditions include: using a secondary mass spectrometry detector, an electrospray ionization source, positive ion multiple reaction monitoring mode, (7aS,2'S)-2-oxo-clopidogrel m/z 338.100→155.000, internal standard m/z 341.100→158.000, and a collision energy of 18-22 eV, preferably 20 eV.

In some embodiments of the disclosure, the protein precipitant comprises methanol and/or acetonitrile;
preferably, in step S2, the protein precipitant of 1-6 times, more preferably 3 times the volume of plasma is added.

In some embodiments of the disclosure, the internal standard working solution is a solution of (7aS,2'S)-2-oxo-clopidogrel-d₃ in methanol.

Preferably, the concentration of the internal standard working solution is 10-50 ng/ml, preferably 20 ng/ml.

Preferably, 1 to 10 µl, preferably 5 µl of the internal standard working solution is added per 100 µl of plasma.

In some embodiments of the disclosure, the volume concentration of the formic acid solution is 0.05% to 0.2%.

In some embodiments of the disclosure, the gradient elution is carried out as specified below:
0 min, 10% A, 90% B;
2 min, 55% A, 45% B;
5 min, 55% A, 45% B;
6 min, 90% A, 10% B;
8 min, 90% A, 10% B;
8.5 min, 10% A, 90% B;
10 min, 10% A, 90% B.

In some embodiments of the disclosure, the flow rate of the mobile phase is 0.1-0.4 ml/min, preferably 0.2 ml/min; the column temperature is 30 to 50°C, preferably 40°C.

In some embodiments of the disclosure, the process of S1 is operated under low temperature condition, preferably on ice.

In some embodiments of the disclosure, the mass spectrometer conditions include a capillary voltage of 4.0KV, a cone voltage of 25V, a desolvation gas temperature of 400°C and a source temperature of 150°C.

The chemical names corresponding to the English abbreviations of the disclosure are as follows:
DTT: dithiothreitol;
TCEP: tris(2-carboxyethyl)phosphine;
ME: 2-mercaptoethanol.

Compared with the prior art, the disclosure has the following beneficial effects.

The method of the disclosure is simple, easy to operate, and has good stability, specificity and sensitivity, and accurate determination results.

Pre-treatment is simple in the disclosure: the sample is obtained by precipitating protein by acetonitrile or methanol, vortexing and centrifuging, and then taking the supernatant. This process is simple and easy, and is suitable for routine quantitative analysis and detection.

The disclosure effectively improves the stability of (7aS,2'S)-2-oxo-clopidogrel in plasma by adding a reducing agent to the plasma. The method of the disclosure has a high selectivity and endogenous substances in the blank plasma do not interfere with the determination of the drug and the internal standard.

The method of the disclosure has a high sensitivity, in which the detection sensitivity of the determination is significantly improved by secondary mass spectrometry detection, with a minimum limit of quantification of 0.03 ng/ml.

In the method of the disclosure, (7aS,2'S)-2-oxo-clopidogrel is in the linear range of 0.03-15 ng/ml with a large span, which can well meet the determination of dynamic analysis changes of drugs in vivo.

The recovery rate of the method of the disclosure is stable, and the within-run and between-run precisions (relative standard deviation, RSD) are both less than 15%.

### Brief Description of the Drawings

Fig. 1 is an MRM plot of the blank control solution in Example 2;
Fig. 2 is an MRM plot of the sample of blank plasma + (7aS,2'S)-2-oxo-clopidogrel in Example 2;
Fig. 3 is an MRM plot of the sample of blank plasma + internal standard in Example 2; and
Fig. 4 is an MRM plot of the sample to be determined in Example 2.

### Detailed Description of Preferred Embodiments

In order to make the purpose, technical solutions and advantages of the examples of the disclosure clearer, the technical solutions in the examples of the disclosure will be described clearly and completely below. Where specific conditions are not indicated in the examples, they are in accordance with conventional conditions or conditions recommended by the manufacturer. Where no manufacturer is indicated for the reagents or instruments used, they are conventional products that are commercially available.

In the examples of the disclosure, the liquid chromatograph is a SHIMADZU Nexera UHPLC LC-30A ultra-high performance liquid chromatograph (Shimadzu, Japan), and the mass spectrometer is a SCIEX Triple Quad^{™} 5500⁺ triple quadruple tandem mass spectrometer equipped with an electrospray ionization source (ESI source, SCIEX, USA).

(7aS,2'S)-2-oxo-clopidogrel (99.9% purity), (7aS,2'S)-2-oxo-clopidogrel-d₃ (99.5% purity) are obtained from Chengdu Shibeikang Biomedical Science and Technology Co Ltd; acetonitrile and methanol are chromatographic grade; all other chemical reagents are analytical pure.

### Example 1

This example discloses a method for determining (7aS,2'S)-2-oxo-clopidogrel in plasma by LC/MS/MS of the disclosure, comprising the following steps:
S1. Whole blood treatment: taking 3 ml of human whole blood anticoagulated with EDTA-K2 after oral administration of (7aS,2'S)-2-oxo-clopidogrel, cooling to 4°C on ice, then adding thereto 60 µl of tris(2-carboxyethyl)phosphine at 100 mg/ml; shaking well and then centrifuging (2-8°C, 3000 rpm for 10 min), and collecting plasma for later use.
S2. Plasma sample pre-treatment: taking 100 µl of the plasma, adding 5 µl of an internal standard working solution and 300 µl of acetonitrile respectively; after vortex mixing, centrifuging at 2-8°C and 10,000 r/min for 10 min, and collecting the supernatant for later use.

Herein, the internal standard working solution is a methanol solution of (7aS,2S)(7aS,2'S)-2-oxo-clopidogrel-d₃ at a concentration of 20 ng/ml.

S3. LC-MS/MS analysis: injecting 10 µl of the supernatant prepared from step S2 for determination, where the liquid chromatography conditions are:
the chromatographic column is Kinetex^{®} 1.7µm C18 100 A 100×2.1 mm; the column temperature is 40°C; the mobile phase is acetonitrile-0.2% aqueous formic acid solution with a flow rate of 0.2 ml/min; and a gradient elution is carried out as specified in the table below:

**Table 1. Gradient Elution**

| Time | Acetonitrile | Aqueous formic acid solution |
|---|---|---|
| 0 min | 10% | 90% |
| 2 min | 55% | 45% |
| 5 min | 55% | 45% |
| 6 min | 90% | 10% |
| 8 min | 90% | 10% |
| 8.5min | 10% | 90% |
| 10.0 min | 10% | 90% |

### Mass spectrometry conditions

ESI ion source, positive ion detection in multiple reaction monitoring mode (MRM); the quantitatively analyzed ions (7aS,2'S)-2-oxo-clopidogrel: m/z 338.100→155.000, IS: m/z 341.100→158.000, a capillary voltage of 4.0 KV, a cone voltage of 25 V, a desolvation gas temperature of 400°C and a source temperature of 150°C. The optimal collision energy is 20 eV.

The above chromatographic conditions are typical conditions. In actual applications, each of the parameters can be appropriately adjusted according to the different characteristics of the instruments used, to obtain the best results.

### Example 2 - Investigation of specificity

This example discloses a specificity test of the method of the disclosure.

### 1. Preparation of a blank control solution:

100 µl of blank plasma without (7aS,2'S)-2-oxo-clopidogrel was taken, and 300 µl of acetonitrile was added; after vortex mixing, the plasma was centrifuged at 2-8°C and 10,000 r/min for 10 min, and the supernatant was collected to produce the blank control solution.

### 2. Preparation of a sample of blank plasma + (7aS,2'S)-2-oxo-clopidogrel:

Blank plasma was taken and (7aS,2'S)-2-oxo-clopidogrel was added to prepare the plasma containing (7aS,2'S)-2-oxo-clopidogrel at a concentration of 0.03 ng/ml; 100 µl of the plasma was then taken and 300 µl of acetonitrile was added; after vortex mixing, the plasma was centrifuged at 2-8°C and 10,000 r/min for 10 min, and the supernatant was collected to produce the sample of blank plasma + (7aS,2'S)-2-oxo-clopidogrel.

### 3. Preparation of a sample of blank plasma + internal standard:

100 µl of blank plasma was taken, and 5 µl of an internal standard working solution and 300 µl of acetonitrile were added respectively; after vortex mixing, the plasma was centrifuged at 2-8°C and 10,000 r/min for 10 min, and the supernatant was collected to produce the sample of blank plasma + internal standard.

Herein, the internal standard working solution was a methanol solution of (7aS,2S)(7aS,2'S)-2-oxo-clopidogrel-d₃ at a concentration of 20 ng/ml.

### 4. Preparation of a sample to be determined:

3 ml of human whole blood anticoagulated with EDTA-K2 after oral administration of (7aS,2'S)-2-oxo-clopidogrel was taken and operated according to S1 and S2 of Example 1, to prepare the sample to be determined.

### 5. LC/MS/MS analysis

10 µl of each of the above blank control solution, the sample of blank plasma + (7aS,2'S)-2-oxo-clopidogrel, the sample of blank plasma + internal standard, and the sample to be determined was taken and injected for determination, respectively. The liquid chromatography conditions and mass spectrometry conditions were the same as in Example 1.

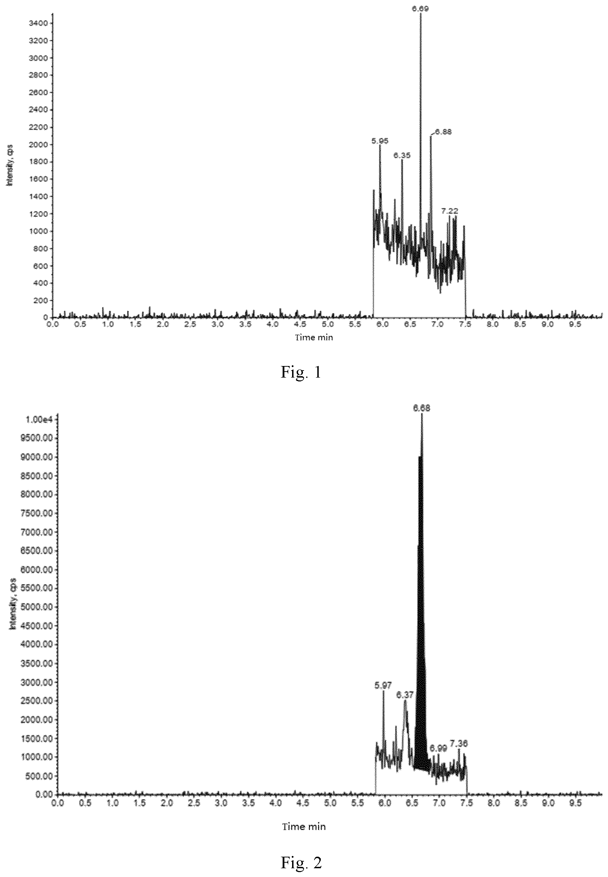

The results are shown in the Figs. 1-4. The results indicate that the method of the disclosure has good specificity, and the endogenous substances in the blank plasma do not interfere with the determination of (7aS,2'S)-2-oxo-clopidogrel and the internal standard.

### Example 3 - Drawing a standard curve

Blank plasma was taken and operated according to S1 of Example 1 to obtain a blank plasma containing the reducing agent; subsequently, standard series solution of (7aS,2'S)-2-oxo-clopidogrel was added to prepare standard plasma containing (7aS,2'S)-2-oxo-clopidogrel at a concentration of 0.03, 0.06, 0.15, 0.75, 3, 7.5, and 15 ng/ml. Herein, the standard series solution of (7aS,2'S)-2-oxo-clopidogrel was made by dissolving (7aS,2'S)-2-oxo-clopidogrel in acetonitrile.

100 µl of the above standard plasma was taken and pre-treated according to the method of S2 of Example 1, and then subjected to LC/MS/MS determination according to the method of S3.

The standard curve is obtained by regression analysis using weighted least squares method. The standard curve equation is: y=0.3909x+0.0701 (r=0.9987), where y represents the ratio of peak area of the drug to be determined to the internal standard, and x represents the concentration of the drug to be determined.

The results show that the linear range for the quantitative determination of (7aS,2'S)-2-oxo-clopidogrel in the method of the disclosure is 0.03-15 ng/ml, and the lowest limit of quantification is 0.03 ng/ml.

### Example 4 - Screening of reducing agent dosage

Blank plasma was taken and TCEP was added to prepare plasma containing TCEP at different concentrations (10, 50, 100 mg/ml). Subsequently, standard series solution of (7aS,2'S)-2-oxo-clopidogrel was added separately to prepare quality control samples with two concentrations of (7aS,2'S)-2-oxo-clopidogrel (0.1 and 12.0 ng/ml), 3 samples for each concentration. They were placed at room temperature and on ice, respectively, and the plasma samples at different placing time points were taken to examine the stability by pre-treating the plasma samples according to the method of S2 of Example 1, followed by LC/MS/MS determination according to the method of S3. The results are shown in Table 2:

**Table 2. Screen results of dosage of reducing agent TCEP**

| Conditions | Theoretical concentration of (7aS,2'S)-2-oxo-clopidogrel (ng/mL) | Placing condition | Placing time (min) | Measured concentratio n (ng/mL) | Recovery rate (%) |
|---|---|---|---|---|---|
| No TCEP | 0.1 | room temperatur e | 0 | 0.0965 | 96.5 |
| | | | 30 | 0.0782 | 78.2 |
| | | | 60 | 0.0633 | 63.3 |
| | | | 120 | 0.0529 | 52.9 |
| | | on ice | 0 | 0.0965 | 96.5 |
| | | | 30 | 0.0842 | 84.2 |
| | | | 60 | 0.0763 | 76.3 |
| | | | 120 | 0.0622 | 62.2 |
| | 12.0 | room temperatur e | 0 | 12.3 | 102.5 |
| | | | 30 | 9.45 | 78.8 |
| | | | 60 | 8.38 | 69.8 |
| | | | 120 | 6.57 | 54.8 |
| | | on ice | 0 | 12.3 | 102.5 |
| | | | 30 | 10.3 | 85.8 |
| | | | 60 | 9.65 | 80.4 |
| | | | 120 | 7.23 | 60.3 |
| TCEP (10 mg/ml) | 0.1 | room temperatur e | 0 | 0.0965 | 96.5 |
| | | | 30 | 0.0952 | 95.2 |
| | | | 60 | 0.0833 | 83.3 |
| | | | 120 | 0.0729 | 72.9 |
| | | on ice | 0 | 0.0965 | 96.5 |
| | | | 30 | 0.0942 | 94.2 |
| | | | 60 | 0.0863 | 86.3 |
| | | | 120 | 0.0782 | 78.2 |
| | 12.0 | room temperatur e | 0 | 12.3 | 102.5 |
| | | | 30 | 11.7 | 97.5 |
| | | | 60 | 10.4 | 86.7 |
| | | | 120 | 9.45 | 78.8 |
| | | on ice | 0 | 12.3 | 102.5 |
| | | | 30 | 11.9 | 99.2 |
| | | | 60 | 10.65 | 88.8 |
| | | | 120 | 9.92 | 82.7 |
| TCEP (50 mg/ml) | 0.1 | room temperatur e | 0 | 0.0965 | 96.5 |
| | | | 30 | 0.0935 | 93.5 |
| | | | 60 | 0.0914 | 91.4 |
| | | | 120 | 0.0895 | 89.5 |
| | | on ice | 0 | 0.0965 | 96.5 |
| | | | 30 | 0.0956 | 95.6 |
| | | | 60 | 0.0932 | 93.2 |
| | | | 120 | 0.0911 | 91.1 |
| | 12.0 | room temperatur e | 0 | 12.3 | 102.5 |
| | | | 30 | 12.1 | 100.8 |
| | | | 60 | 11.9 | 99.2 |
| | | | 120 | 10.6 | 88.3 |
| | | on ice | 0 | 12.3 | 102.5 |
| | | | 30 | 12.2 | 101.7 |
| | | | 60 | 11.9 | 99.2 |
| | | | 120 | 11.4 | 95.0 |
| TCEP (100 mg/ml) | 0.1 | room temperatur e | 0 | 0.0965 | 96.5 |
| | | | 30 | 0.0955 | 95.5 |
| | | | 60 | 0.0934 | 93.4 |
| | | | 120 | 0.0915 | 91.5 |
| | | on ice | 0 | 0.0965 | 96.5 |
| | | | 30 | 0.0958 | 95.8 |
| | | | 60 | 0.0942 | 94.2 |
| | | | 120 | 0.0951 | 95.1 |
| | 12.0 | room temperatur e | 0 | 12.3 | 102.5 |
| | | | 30 | 12.2 | 101.7 |
| | | | 60 | 11.9 | 99.2 |
| | | | 120 | 11.6 | 96.7 |
| | | on ice | 0 | 12.3 | 102.5 |
| | | | 30 | 12.4 | 103.3 |
| | | | 60 | 12.1 | 100.8 |
| | | | 120 | 11.8 | 98.3 |

As shown in Table 2, (7aS,2'S)-2-oxo-clopidogrel in the plasma samples without TCEP degraded fast, and (7aS,2'S)-2-oxo-clopidogrel had a good stability when TCEP was added at a concentration of 50 mg/ml or more. Meanwhile, (7aS,2'S)-2-oxo-clopidogrel plasma samples were more stable when placed on ice than at room temperature.

### Example 5 - Screening of reducing agent type

Blank plasma was taken and different antioxidants (DTT, TCEP, ME) were added to prepare plasma containing an antioxidant at a concentration of 100 mg/ml. Subsequently, standard series solution of (7aS,2'S)-2-oxo-clopidogrel was added separately to prepare quality control samples with two concentrations of (7aS,2'S)-2-oxo-clopidogrel (0.1 and 12.0 ng/ml), 3 samples for each concentration. They were placed in a refrigerator at -80°C, and the plasma samples at different placing time points were taken to examine the stability by pre-treating the plasma samples according to the method of S2 of Example 1, followed by LC/MS/MS determination according to the method of S3. The results are shown in Table 3:

**Table 3. Screen results of reducing agent type**

| Reducing agent type | Theoretical concentration (ng/mL) | Placing time (day) | Measured value (ng/mL) | Recovery rate (%) |
|---|---|---|---|---|
| DTT | 0.1 | 0 | 0.1022 | 102.2 |
| | | 10 | 0.1033 | 103.3 |
| | | 30 | 0.0955 | 95.5 |
| | | 60 | 0.0899 | 89.9 |
| | 12.0 | 0 | 12.1435 | 101.2 |
| | | 10 | 12.0024 | 100.0 |
| | | 30 | 11.8674 | 98.9 |
| | | 60 | 11.0928 | 92.4 |
| TCEP | 0.1 | 0 | 0.1022 | 102.2 |
| | | 10 | 0.1053 | 105.3 |
| | | 30 | 0.0993 | 99.3 |
| | | 60 | 0.1008 | 100.8 |
| | 12.0 | 0 | 12.1435 | 101.2 |
| | | 10 | 12.0566 | 100.5 |
| | | 30 | 12.2674 | 102.2 |
| | | 60 | 12.0928 | 100.8 |
| ME | 0.1 | 0 | 0.1022 | 102.2 |
| | | 10 | 0.1006 | 100.6 |
| | | 30 | 0.0915 | 91.5 |
| | | 60 | 0.0866 | 86.6 |
| | 12.0 | 0 | 12.1435 | 101.2 |
| | | 10 | 11.9024 | 99.2 |
| | | 30 | 11.5744 | 96.5 |
| | | 60 | 10.5488 | 87.9 |

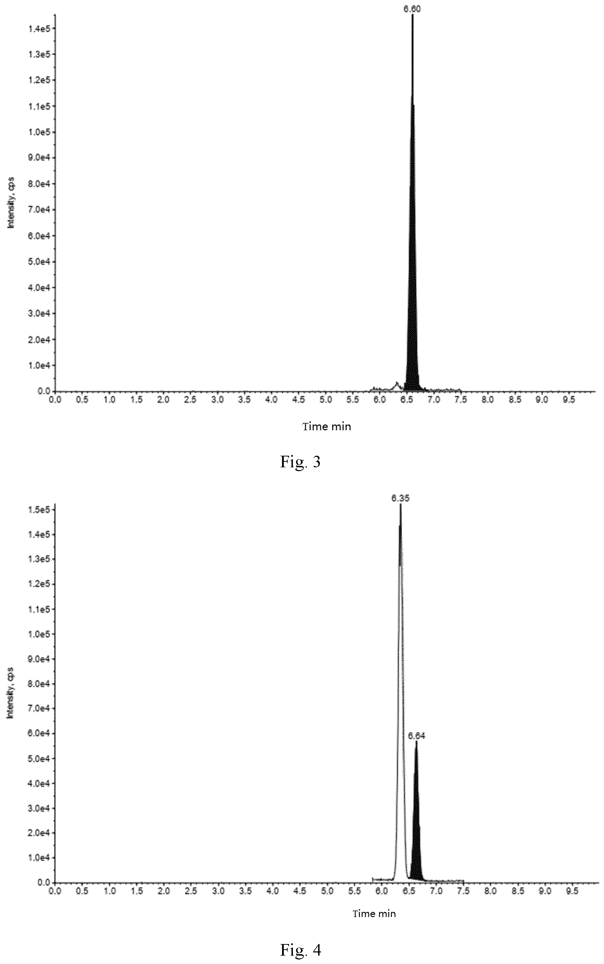

As shown in Fig. 3, the stability of the plasma samples with the reducing agent was better than that of the plasma samples without the reducing agent, and the stability of the plasma samples with TCEP was better than that of the plasma samples with DTT and ME. This indicates that the addition of the reducing agent to the plasma samples can significantly enhance the stability of the samples, and the antioxidant effect of TCEP is significantly better than that of DTT and ME.

### Example 6 - Investigation of extraction recovery rate

Blank plasma was taken and operated according to S1 of Example 1 to obtain a blank plasma containing the reducing agent. Subsequently, standard series solution of (7aS,2'S)-2-oxo-clopidogrel was added separately to prepare quality control samples containing (7aS,2' S)-2-oxo-clopidogrel at concentrations of 0.1, 5 and 12 ng/ml. For each concentration, 6 copies were prepared in parallel. 100 µl of each of the above quality control samples was taken and pre-treated according to the method of S2 of Example 1 to obtain a supernatant with concentrations of 0.025, 1.235, and 2.96 ng/ml, and the supernatant was taken and subjected to LC/MS/MS determination according to the method of S3 of Example 1.

(7aS,2'S)-2-oxo-clopidogrel was taken separately and acetonitrile was added to prepare solution with concentrations of 0.025, 1.235, and 2.96 ng/ml, respectively. 10 µl of each of the solution was taken and subjected to LC/MS/MS determination according to the method of S3 of Example 1.

The extraction recovery rate of the samples was examined as the ratio of the peak area of the plasma sample to that of the acetonitrile solution of (7aS,2'S)-2-oxo-clopidogrel at the same concentration. The results of analysis and determination are shown in Table 4:

**Table 4. Extraction recovery rate of (7aS,2'S)-2-oxo-clopidogrel (n=6)**

| Concentration (ng/ml) | Recovery rate (%) | Average value (%) | RSD (%) |
|---|---|---|---|
| 0.1 | 97.20 | 99.34 | 3.70 |
| | 106.15 | | |
| | 100.88 | | |
| | 97.25 | | |
| | 98.12 | | |
| | 96.46 | | |
| 5 | 99.59 | 103.18 | 2.34 |
| | 101.78 | | |
| | 102.78 | | |
| | 106.68 | | |
| | 103.81 | | |
| | 104.43 | | |
| 12 | 105.63 | 104.07 | 2.04 |
| | 101.01 | | |
| | 105.79 | | |
| | 104.26 | | |
| | 105.82 | | |
| | 101.89 | | |

As shown in Table 4, the extraction recovery rates of (7aS,2'S)-2-oxo-clopidogrel at the three concentrations were 99.34%, 103.18% and 104.07%, respectively, indicating that the method of the disclosure has a good extraction recovery rate.

### Example 7 - Investigation of precision and accuracy

Blank plasma was taken and operated according to S1 of Example 1 to obtain a blank plasma containing the reducing agent. Subsequently, standard series solution of (7aS,2'S)-2-oxo-clopidogrel was added to prepare quality control samples containing (7aS,2'S)-2-oxo-clopidogrel at concentrations of 0.1, 5 and 12 ng/ml, which were then pre-treated. For each concentration, 6 copies were prepared in parallel.

Three independent analytical runs were determined in duplicate on different days. Accuracy describes the closeness of the measured concentration of the analytical method to the theoretical concentration, expressed as (measured concentration/theoretical concentration) × 100%. Precision describes the closeness between duplicate measurements of an analyte, expressed as RSD. The analysis results are shown in Table 5:

**Table 5. Accuracy and precision of the method for determining (7aS,2'S)-2-oxo-clopidogrel in plasma samples**

| Concentration (ng/ml) | Analytical run | Accuracy (%) | Within-run RSD (%) | Between-run RSD (%) |
|---|---|---|---|---|
| 0.1 | A | 91.33 | 10.61 | 6.30 |
| | B | 94.33 | 3.40 | |
| | C | 103.00 | 5.32 | |
| 5 | A | 86.79 | 1.79 | 4.07 |
| | B | 89.49 | 1.48 | |
| | C | 94.05 | 2.27 | |
| 12 | A | 87.33 | 2.74 | 5.96 |
| | B | 90.60 | 1.70 | |
| | C | 98.36 | 2.46 | |

As shown in Table 5, the accuracy of (7aS,2'S)-2-oxo-clopidogrel is in the range of 86.79-103.00%, the within-run RSD is in the range of 1.48-10.61%, and the between-run RSD is in the range of 4.07-6.30%, indicating good accuracy and precision of this method.

### Example 8 - Investigation of stability

Blank plasma was taken and operated according to S1 of Example 1 to obtain a blank plasma containing the reducing agent. Subsequently, a standard series solution of (7aS,2'S)-2-oxo-clopidogrel was added to prepare quality control samples containing (7aS,2'S)-2-oxo-clopidogrel at concentrations of 0.1 and 12 ng/ml. The plasma samples were examined for stability after ice box short-term placement, stability after room temperature short-term placement, stability after freeze-thaw cycle and stability after long-term cryopreservation.

The plasma samples at different placing time points were taken to examine the stability by pre-treating the plasma samples according to the method of S2 of Example 1, followed by LC/MS/MS determination according to the method of S3. The results are shown in Table 6:

**Table 6. Stability results of (7aS,2'S)-2-oxo-clopidogrel in plasma**

| Conditions | Theoretical concentration (ng/mL) | Measured concentration (ng/mL) | Average measured concentration (ng/mL) | Recovery rate (%) |
|---|---|---|---|---|
| Placing in ice box for 4 h | 0.1 | 0.0993 | 0.1000 | 100.0 |
| | | 0.1020 | | |
| | | 0.1013 | | |
| | | 0.0960 | | |
| | | 0.1013 | | |
| | 12.0 | 12.0320 | 11.8184 | 98.5 |
| | | 11.7632 | | |
| | | 11.6883 | | |
| | | 11.8822 | | |
| | | 11.7261 | | |
| Placing at room temperature for 1 h | 0.1 | 0.1013 | 0.1021 | 102.1 |
| | | 0.1027 | | |
| | | 0.1020 | | |
| | | 0.1040 | | |
| | | 0.1007 | | |
| | 12.0 | 11.8349 | 11.9269 | 99.4 |
| | | 11.7245 | | |
| | | 11.8592 | | |
| | | 12.1616 | | |
| | | 12.0541 | | |
| three cycles of freeze-thaw | 0.1 | 0.1007 | 0.1003 | 100.3 |
| | | 0.1013 | | |
| | | 0.1013 | | |
| | | 0.1007 | | |
| | | 0.0973 | | |
| | 12.0 | 11.9123 | 11.9191 | 99.3 |
| | | 11.8416 | | |
| | | 11.9078 | | |
| | | 11.8995 | | |
| | | 12.0342 | | |
| Placing below - 60 °C for 36 days | 0.1 | 0.0980 | 0.0964 | 96.4 |
| | | 0.0940 | | |
| | | 0.0987 | | |
| | | 0.0980 | | |
| | | 0.0933 | | |
| | 12.0 | 11.6573 | 11.7299 | 97.7 |
| | | 11.9738 | | |
| | | 11.7638 | | |
| | | 11.8026 | | |
| | | 11.4518 | | |

As shown in Table 6, for the plasma samples of (7aS,2'S)-2-oxo-clopidogrel (low concentration quality control and high concentration quality control), the recovery rates after placing in ice box for 4 h are 100.0% and 98.5%, respectively; the recovery rates after placing at room temperature for 1 h are 102.1% and 99.4%, respectively; the recovery rates after three cycles of freeze-thaw are 100.3% and 99.3%, respectively; and the recovery rates after placing below -60 °C for 36 days are 96.4% and 97.7%, respectively, indicating a good stability of the method.

The above examples are only preferred embodiments of the disclosure and should not be used to limit the scope of protection of the disclosure. Any unsubstantial changes or modifications made in the main design idea and spirit of the disclosure, aiming to solve the technical problems consistent with the disclosure, shall be included in the protection scope of the disclosure.

## Claims

1. A method for treating a plasma sample containing (7aS,2'S)-2-oxo-clopidogrel, comprising: taking whole blood containing (7aS,2'S)-2-oxo-clopidogrel, adding a reducing agent, and collecting plasma.

2. The method according to claim 1, wherein the reducing agent is a protein reducing agent; preferably, the protein reducing agent is at least one of dithiothreitol, tris(2-carboxyethyl)phosphine, and 2-mercaptoethanol, more preferably tris(2-carboxyethyl)phosphine;
preferably, the concentration of the reducing agent in the plasma is 10-100 mg/ml.

3. A method for determining (7aS,2' S)-2-oxo-clopidogrel in plasma, comprising treating a sample to be determined, by using the method according to claim 1 or 2.

4. The method for determining (7aS,2'S)-2-oxo-clopidogrel in plasma according to claim 3, wherein LC/MS/MS is used for determination, and the method includes the following steps:
S1. Whole blood treatment: taking whole blood, adding a reducing agent solution, centrifuging, and collecting plasma;
S2. Plasma sample pre-treatment: adding a protein precipitant and an internal standard working solution to the plasma, vortexing and then centrifuging, and taking the supernatant;
S3. LC/MS/MS determination: injecting the supernatant obtained from step S2 into an ultra-high performance liquid chromatography tandem mass spectrometry for determination,
wherein the liquid chromatography conditions include: a chromatographic column with octadecylsilane bonded silica gel packing, acetonitrile as mobile phase A and aqueous formic acid solution as mobile phase B, and a gradient elution is carried out as specified below:
0 min, 5-15% A, 85-95% B;
2 min, 50-60% A, 40-50% B;
5 min, 50-60% A, 40-50% B;
6 min, 85-95% A, 5-15% B;
8 min, 85-95% A, 5-15% B;
8.5 min, 5-15% A, 85-95% B;
10 min, 5-15% A, 85-95% B;
wherein the mass spectrometry conditions include: using a secondary mass spectrometry detector, an electrospray ionization source, positive ion multiple reaction monitoring mode, (7aS,2'S)-2-oxo-clopidogrel m/z 338.100→155.000, internal standard m/z 341.100→158.000, and a collision energy of 18-22 eV, preferably 20 eV.

5. The method for determining (7aS,2'S)-2-oxo-clopidogrel in plasma according to claim 4, wherein the protein precipitant comprises methanol and/or acetonitrile;
preferably, in step S2, the protein precipitant of 1-6 times, more preferably 3 times the volume of plasma is added.

6. The method for determining (7aS,2'S)-2-oxo-clopidogrel in plasma according to claim 5, wherein the internal standard working solution is a solution of (7aS,2'S)-2-oxo-clopidogrel-d₃ in methanol;
preferably, the concentration of the internal standard working solution is 10-50 ng/ml;
preferably, 1 to 10 µl of the internal standard working solution is added per 100 µl of plasma; preferably, the volume concentration of the formic acid solution is 0.05% to 0.2%.

7. The method for determining (7aS,2'S)-2-oxo-clopidogrel in plasma according to claim 4, wherein the gradient elution is carried out as specified below:
0 min, 10% A, 90% B;
2 min, 55% A, 45% B;
5 min, 55% A, 45% B;
6 min, 90% A, 10% B;
8 min, 90% A, 10% B;
8.5 min, 10% A, 90% B;
10 min, 10% A, 90% B.

8. The method for determining (7aS,2'S)-2-oxo-clopidogrel in plasma according to claim 7, wherein the flow rate of the mobile phase is 0.1-0.4 ml/min, preferably 0.2 ml/min; the column temperature is 30 to 50°C, preferably 40°C.

9. The method according to claim 4, wherein the process of S1 is operated under low temperature condition, preferably on ice.

10. The method according to claim 4, wherein the desolvation gas temperature and the source temperature are 400°C and 150°C, respectively.
